(19) Europäisches Patentamt
European Patent Office
Office européen des brevets

(11) **EP 4 787 385 A1**

(12) **EUROPEAN PATENT APPLICATION**

(43) Date of publication:
**05.08.2026 Bulletin 2026/32**

(21) Application number: **26150502.8**

(22) Date of filing: **07.01.2026**

(51) International Patent Classification (IPC):
**G16H 50/30** (2018.01)   **G16H 50/20** (2018.01)
**A61B 5/00** (2006.01)   **G06N 3/0442** (2023.01)
**G16H 50/70** (2018.01)

(52) Cooperative Patent Classification (CPC):
**G16H 50/30; A61B 5/7267; G06N 3/044;
G06N 3/0442; G06N 3/045; G06N 3/08;
G16H 50/20; G16H 50/70**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **31.01.2025 US 202519042102**

(71) Applicant: **GE Precision Healthcare LLC
Waukesha, WI 53188 (US)**

(72) Inventors:
• **MANICKAM, Kalaivani
Waukesha, 53188 (US)**
• **JONNADULA, BharathKumar
Waukesha, 53188 (US)**
• **BOGINENI, Kiran
Waukesha, 53188 (US)**

(74) Representative: **Kilburn & Strode LLP
Lacon London
84 Theobalds Road
Holborn
London WC1X 8NL (GB)**

(54)   **PRE-TERM INFANT VIABILITY SCORING**

(57)   Some embodiments of the present disclosure include a processor and a memory. The memory includes instructions executable by the processor to receive training data having vitals scores for pre-term infants indicating a healthiness of a pre-term infant having specific vitals measurements. Further, the instructions are executable to receive training data having maternal health parameters and pregnancy parameters for pregnant women that gave birth to pre-term infants. Additionally, the instructions are executable to receive training data having pre-term infant health parameters and birth parameters for the pre-term infants. Further, the instructions are executable to train an artificial intelligence model to infer a viability score by using the maternal parameters training data, pre-term parameters training data, pre-term vitals training data, and supervised learning. Additionally, the viability score indicates a likelihood of survival for a pre-term infant having a vitals score, maternal parameters, and pre-term parameters.

FIG. 1

**Description**

BACKGROUND

The present disclosure generally relates to pre-term infant viability, and specifically to pre-term infant viability scoring.

Advances in intensive care have lowered the gestational age at which pre-term infants can survive. However, those born at the lowest gestational ages face a higher risk of death and poor neurological outcomes compared to those born later. These "peri-viable" pre-term infants, typically born between 22 to 32 weeks of gestation, may receive active care that ranges from palliative care to assertive intensive care, depending on decisions made by parents and healthcare professionals (HCPs). Active care refers to the class of care given to pre-term infants by the neonatal intensive care unit (NICU) clinicians, such as, resuscitation during the first few weeks after birth.

SUMMARY

This Summary is provided to introduce a selection of concepts that are further described below in the Detailed Description. This Summary is not intended to identify key or essential features of the claimed subject matter, nor is it intended to be used as an aid in limiting the scope of the claimed subject matter.

In one aspect of the disclosure, a system includes a processor and a memory. The memory includes instructions executable by the processor to receive training data having vitals scores for pre-term infants indicating a healthiness of a pre-term infant having specific vitals measurements. Further, the instructions are executable to receive training data having maternal health parameters and pregnancy parameters for pregnant women that gave birth to pre-term infants. Additionally, the instructions are executable to receive training data having pre-term infant health parameters and birth parameters for the pre-term infants. Further, the instructions are executable to train an artificial intelligence model to infer a viability score by using the maternal parameters training data, pre-term parameters training data, pre-term vitals training data, and supervised learning. Additionally, the viability score indicates a likelihood of survival for a pre-term infant having a vitals score, maternal parameters, and pre-term parameters.

In one embodiment, the viability score further indicates a likelihood of morbidity and mortality.

In one embodiment, the instructions executable by the processor to receive a pre-term vitals training dataset having vitals measurements for pre-term infants. Additionally, the instructions are executable by the processor to perform linear processing on the pre-term vitals training dataset. Further, , the instructions are executable by the processor to perform non-linear processing on the pre-term vitals training dataset. Additionally, the instructions are executable by the processor to perform wavelet processing on the pre-term vitals training dataset. Further, the instructions are executable by the processor to train a recurrent neural network model to infer a vitals score by using the pre-term vitals training dataset and supervised learning. Additionally, the vitals score indicates a healthiness of a pre-term infant having specific vitals measurements.

In one embodiment, the recurrent neural network model includes a bidirectional long short-term memory model.

In one embodiment, the instructions are executable by the processor to receive a heart rate measurements that are measured over a specific period of time. Additionally, the instructions are executable by the processor to receive oxygen saturation measurements that are measured over the specific period of time or continuously. Further, the instructions are executable by the processor to receive respiration rate measurements that are measured over the specific period of time or continuously. Additionally, the instructions are executable by the processor to receive body temperature measurements that are measured over the specific period of time or continuously. Further, the instructions are executable by the processor to perform linear processing on the heart rate measurements, oxygen saturation measurements, respiration rate measurements, and body temperature measurements. Additionally, the instructions are executable by the processor to perform non-linear processing on the heart rate measurements, oxygen saturation measurements, respiration rate measurements, and body temperature measurements. Further, the instructions are executable by the processor to perform wavelet processing on the heart rate measurements, oxygen saturation measurements, respiration rate measurements, and body temperature measurements. Additionally, the instructions are executable by the processor to infer a real-time vitals score for the pre-term infant using the trained deep learning model having inputs including: the heart rate measurements, the oxygen saturation measurements, the respiration rate measurements, and the body temperature measurements.

In one embodiment, the instructions are executable by the processor to receive the real-time vitals score for the pre-term infant. Additionally, the instructions are executable by the processor to receive maternal parameters including individual maternal health parameters, and individual pregnancy parameters for a mother that gave birth to the pre-term infant. Further, the instructions are executable by the processor to receive pre-term infant parameters including individual pre-term health parameters, and individual birth parameters for the pre-term infant. Additionally, the instructions are executable by the processor to infer a viability score for the pre-term infant using the machine learning model having inputs including: the real-time vitals score, the maternal parameters, and the pre-term infant parameters.

In one embodiment, the instructions are executable by the processor to present the viability score in real-time using an electronic device.

In one aspect of the disclosure, a method includes receiving a vitals score for a pre-term infant, the vitals score indicating a healthiness of the pre-term infant having specific vitals measurements. Additionally, the method includes receiving maternal health parameters and pregnancy parameters for a mother that gave birth to the pre-term infant. Further, the method includes receiving pre-term infant health parameters and birth parameters for the pre-term infant. Additionally, the method includes inferring a viability score for the pre-term infant by using a machine learning model trained using a maternal parameters training dataset, a pre-term parameters training dataset, a pre-term vitals training dataset, and supervised learning. Further, the machine learning model includes inputs comprising the vitals score, the maternal health parameters, and the pre-term infant health parameters. Additionally, the viability score indicates a likelihood of survival for a pre-term infant having the vitals score, the maternal health parameters, and the pre-term infant health parameters. Further, the pre-term vitals training dataset includes training vitals scores for pre-term infants. Additionally, the maternal parameters training dataset includes maternal health parameters and pregnancy parameters for pregnant women that gave birth to the pre-term infants. Further, the pre-term parameters training dataset includes pre-term infant health parameters and birth parameters for the pre-term infants.

In one embodiment, the method includes receiving heart rate measurements that are measured over a specific period of time. Further, the method includes receiving oxygen saturation measurements that are measured over the specific period of time. Additionally, the method includes receiving respiration rate measurements that are measured over the specific period of time. Further, the method includes receiving body temperature measurements that are measured over the specific period of time. Additionally, the method includes performing linear processing on the heart rate measurements, oxygen saturation measurements, respiration rate measurements, and body temperature measurements. Further, the method includes performing non-linear processing on the heart rate measurements, oxygen saturation measurements, respiration rate measurements, and body temperature measurements. Additionally, the method includes performing wavelet processing on the heart rate measurements, oxygen saturation measurements, respiration rate measurements, and body temperature measurements. Further, the method includes inferring the vitals score for the pre-term infant by using a recurrent neural network model trained with a pre-term vitals training dataset comprising vitals measurements for pre-term infants. Additionally, the recurrent neural network model has inputs including the heart rate measurements, the oxygen saturation measurements, respiration rate measurements, and the body temperature measurements.

In one aspect of the present disclosure, a computer-readable storage medium includes instructions that are executable by a processor to receive a vitals score for a pre-term infant. Additionally, the vitals score indicates a healthiness of a pre-term infant having specific vitals measurements. Further, the instructions are executable by the processor to receive maternal health parameters and pregnancy parameters for a mother that gave birth to the pre-term infant. Additionally, the instructions are executable by the processor to receive pre-term infant health parameters and birth parameters for the pre-term infant. Further, the instructions are executable by the processor to infer a viability score for the pre-term infant by using a machine learning model trained using a maternal parameters training dataset, a pre-term parameters training dataset, a pre-term vitals training dataset, and supervised learning. Additionally, the machine learning model includes inputs having the vitals score, the maternal health parameters, and the pre-term infant health parameters. Further, the viability score indicates a likelihood of survival for a pre-term infant having the vitals score, the maternal health parameters, and the pre-term infant health parameters. Additionally, the pre-term vitals training dataset includes training vitals scores for pre-term infants. Further, the maternal parameters training dataset includes maternal health parameters and pregnancy parameters for pregnant women that gave birth to the pre-term infants. Additionally, the pre-term parameters training dataset includes pre-term infant health parameters and birth parameters for the pre-term infants.

Various other features, objects, and advantages of the invention will be made apparent from the following description taken together with the drawings.

BRIEF DESCRIPTION OF THE DRAWINGS

The present disclosure is described with reference to the following Figures.

Fig. 1 is a data flow diagram of a system for pre-term infant viability scoring according to one embodiment of the present disclosure.
Fig. 2 is a data flow diagram of a system for pre-term infant viability scoring according to one embodiment of the present disclosure.
Fig. 3 is an example recurrent neural network according to one embodiment of the present disclosure.
Fig. 4 is a diagram of an example neuron, described with respect to Fig. 3 according to one embodiment of the present disclosure.
Fig. 5 is a diagram of a system for vitals score training on a Bi-LSTM model according to one embodiment of the present disclosure.
Fig. 6 is a data flow diagram of a system for calculating a viability score for pre-term infants according to one embodiment of the present disclosure.

Fig. 7 is a flow chart of a method for pre-term infant viability scoring according to one embodiment of the present disclosure.

Fig. 8 is a flow chart of a method for pre-term infant viability scoring according to one embodiment of the present disclosure.

Fig. 9 is an exemplary pre-term infant viability scoring manager according to one embodiment of the present disclosure.

DETAILED DESCRIPTION

In the present description, certain terms have been used for brevity, clarity and understanding. No unnecessary limitations are to be inferred therefrom beyond the requirement of the prior art because such terms are used for descriptive purposes only and are intended to be broadly construed.

As used herein, unless otherwise limited or defined, discussion of particular directions is provided by example only, with regard to particular embodiments or relevant illustrations. For example, discussion of "top," "bottom," "front," "rear," "left," "right," "horizontal," "vertical," and "longitudinal" features and/or relative motion, e.g., movement "up" and "down," is generally intended as a description only of the orientation of such features relative to a reference frame of a particular example or illustration. Correspondingly, for example, a "top" feature may sometimes be disposed below a "bottom" feature (and so on), in some arrangements or embodiments. Additionally or alternatively, embodiments may be arranged in a different orientation such that "top" and "bottom" features are arranged horizontally relative to each other, for example in a "left-to-right" orientation.

The use herein of the terms "including," "comprising," or "having," and variations thereof, is meant to encompass the elements listed thereafter and equivalents thereof, as well as additional elements. Embodiments recited as "including," "comprising," or "having" certain elements are also contemplated as "consisting essentially of" and "consisting of" those certain elements.

As stated previously, pre-term infants born between 22 and 28 weeks of gestation may be considered, "extreme pre-term," and as such, may receive a range of active care from palliative care to intensive care, depending on decisions made by parents and healthcare professionals. Similarly, pre-term infants born between 28 and 32 weeks of gestation may receive a range of care based on such decisions. More specifically, these decisions may be based on factors like birth weight, gender, and congenital abnormalities. The choice of active care given to the pre-term infants may be decided on in a collaboration between the parents and healthcare professionals. However, the decisions about the care given may be biased due to disability of the pre-term infant, uncertainty of outcome, racism (insidious or otherwise), and ethnic or other prejudices on the part of obstetricians, neonatologists, and/or other healthcare professionals.

Parents often lack the medical knowledge to make informed decisions about the care of pre-term infants and thus, may rely on guidance from HCPs, which includes input from obstetricians, neonatologists, midwives, and nurses. However, studies show that HCPs may have inaccurate perceptions of survival rates for these pre-term infants, with the reasons for these inaccuracies not well understood. However, medical literature recognizes one reason: implicit bias. Implicit bias refers to a subconscious attitude that is shaped by personal background and experiences, which contributes to disparities in care and lower quality care. Further, bias against disability may affect decision-making and counseling, often resulting in negative messaging from HCPs to parents. More specifically, some HCPs may view disability as a burden, and hence, encourage palliative care instead of resuscitation for pre-term infants born before 22 weeks of gestation. Additionally, obstetricians may be more pessimistic about survival prospects than neonatologists. Conversely, pediatricians (who care for children in the long-term) may be more optimistic about the prospects for survival. For at least these reasons, guidance for parents from healthcare professionals may not be reliable, and as such, undermines the decision-making upon which this guidance is based.

Accordingly, some embodiments of the present disclosure may mitigate bias and other factors contributing to inaccuracy by providing an objective viability score, indicating the likelihood of survival of pre-term infants. This objective viability score may be based on empirical data instead of potentially biased opinions. More specifically, such embodiments may derive an objective viability score by using deep learning, and machine learning model networks that are trained to determine viability based on real-time physiological data, personal health data, historical health data, and other empirical data that is relevant to the determination of viability. Accordingly, such models may take into consideration the continuous vital health parameters of the pre-term infant (e.g., heart rate, respiration rate), and other factors such as medical history, and the like. In these ways, such embodiments may provide an objective score that classifies the viability of the pre-term infant on a numeric scale, thus giving parents and HCPs an objective factor to consider when making healthcare decisions for pre-term infants.

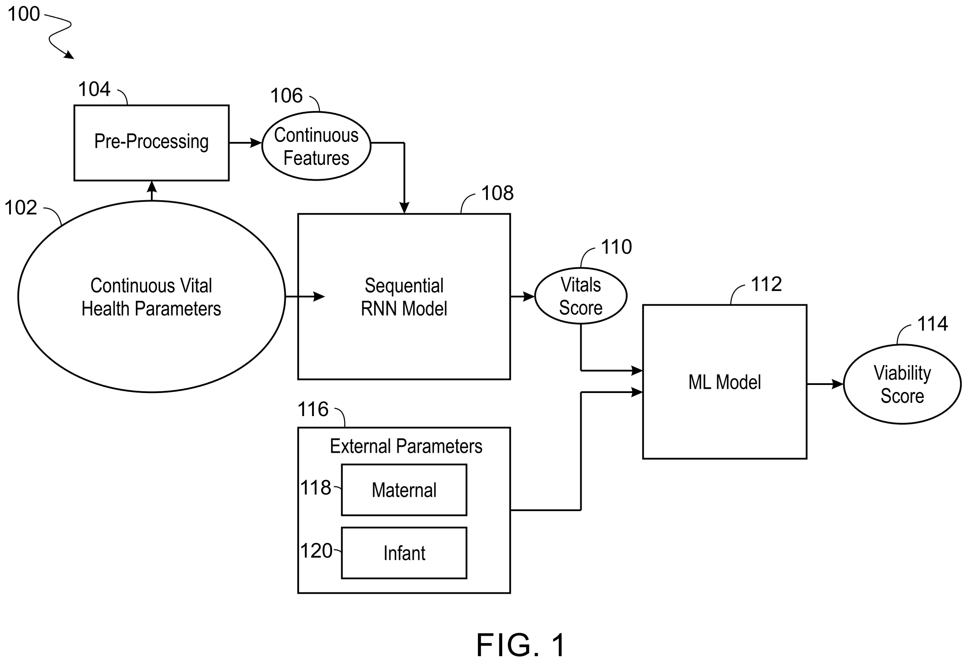

Fig. 1 is a data flow diagram of a system 100 for pre-term infant viability scoring according to one embodiment of the present disclosure. The system 100 may be useful for real-time and offline viability scoring. In the data flow diagram, the circular (e.g., oval) elements represent data, and the quadrilateral (e.g., rectangular) elements represent processes. Further, arrows into a process indicate data inputs, and arrows out of a process indicate data outputs. For example, the system 100 may input continuous vital health parameters 102 to pre-processing 104, which outputs (e.g., calculates) the continuous features 106.

The continuous vital health parameters 102 may include heart rate, oxygen saturation percentage (SP02%), respiration rate, body temperature (core and/or extremity), and the like. The continuous vital health parameters 102 may be measures of physiological characteristics of the pre-term infant over time, thus, not a single numeric value, but rather, a sequence of continuous data, that is useful for analyzing within various time segments, e.g., one hour, two hours, and the like. The length of the time segment for any analysis may be adaptive, i.e., based on characteristics of the continuous vital health parameters 102. For example, if the heart rate (or other continuous vital health parameters 102) exceeds a predetermined variance at shorter intervals of time, the time segment for determining viability may be shorter. In contrast, if one or more of the continuous vital health parameters 102 stays within this predetermined variance at longer intervals, the time segment for determining viability may be longer. The pre-processing 104 may perform a wavelet processing using a convolutional neural network (CNN) model to extract continuous feature sequences. The wavelet processing may perform a wavelet decomposition of the continuous feature sequences into smaller subsequences. Further, the CNN model may extract continuous features 106, which may include features of these subsequences. Additionally, the continuous features 106 may be input to a recurrent neural network (RNN) model 108 for sequential analysis. The pre-processing 104 may also perform other linear and/or non-linear processing to generate continuous features 106 for input to the RNN model 108. Linear processing may generate continuous features 106, such as, mean, variance, skewness, standard deviation, and the like. Additionally, non-linear processing may generate continuous features 106, such as, complexity, entropy, and fractal dimension features, and the like. Further, the continuous vital parameters 102 may be input to the RNN model 108 for sequential processing. In these ways, the RNN model 108 may use the processed signals, e.g., continuous features 106, and the direct vital signals, e.g., continuous vital health parameters 102, individually, or in combination, for sequential processing.

According to some embodiments of the present disclosure, the sequential RNN model 108 may be a deep learning model that performs sequential processing to generate a vitals score 110 through classification or prediction. The vitals score 110 may be a numeric value that provides an indication of the health of the pre-term infant based on the continuous vital health parameters 102 and/or characteristics of these vital health parameters (e.g., continuous features 106). According to one embodiment of the present disclosure, the vitals score 110 may range from 1 to 5, where one indicates a low health score, and five indicates a high health score. According to some embodiments of the present disclosure, the sequential RNN model 108 may be trained using supervised learning. Thus, the training dataset for the sequential RNN model 108 may include training samples of historical data (e.g., continuous vital health parameters 102 and/or continuous features 106) that are labeled with a vitals score 110. According to some embodiments of the present disclosure, a clinician and/or other healthcare professional may label the training samples based on data that is not relevant to the biases referenced herein. Advantageously, training the sequential RNN model 108 in this way makes it possible to avoid introducing bias (implicit or otherwise) into the vitals score 110, which is one factor in determining of the likelihood of survival for pre-term infants. Once trained, the sequential RNN model 108 may determine, for the continuous vital health parameters 102 and the continuous features 106, a probability of the accuracy of each potential vitals score. Alternatively, the vitals score 110 may be an alpha character, ranging from A to F, where A may represent a high health score, and F representing a low health score. In another alternative, the vitals score 110 can be selected from a number of text descriptions indicating the quality of the pre-term infant's health. For example, the vitals score 110 may be one of, "high," "medium," and "low." Alternatively, some embodiments of the present disclosure may associate such textual descriptions with specific ranges of numeric and/or alpha values.

According to some embodiments of the present disclosure, the vitals score 110 may be input with external parameters 116 to a machine learning model 114, which may calculate a viability score 114. The external parameters 116 may include information about the mother and pre-term infant that are relevant to viability. More specifically, the external parameters 116 include maternal parameters 118 and infant parameters 120. The maternal parameters 118 may include the week of gestation when the pre-term infant was born, an obesity indicator, a diabetes indicator, a preeclampsia indicator, and the like. The indicators may be Boolean or ranged values. For example, ranged values for an obesity indicator may include a body mass index. Further, ranged values for a diabetes indicator may include one or more measures of the mother's blood sugar. With respect to the external parameters 116 for the pre-term infant, the infant parameters 120 may include the Apgar score, birth weight, pneumonia indicator, hypoxic-ischemic encephalopathy (HIE) indicator, size indicator, necrotizing enterocolitis indicator (NEC), and the like. The size indicator may be a Boolean value indicating whether the pre-term infant has a lower birth weight and/or length with respect to the gestational week of birth. This determination may be based on a predetermined threshold for birth weight and/or length. Alternatively, the size indicator may be a numeric value indicating the percentage of the birth weight and/or length with respect to the threshold values. Similar to the maternal parameters 118, other infant parameters 120 may also be Boolean or ranged values.

According to some embodiments of the present disclosure, the machine learning model 112 may be trained using supervised learning to generate the viability score 114 based on the vitals score 110 provided by the sequential RNN model 108 and the external parameters 116. In such training, a clinician may label samples of historical data (e.g., external parameters 116) with viability scores 114. Additionally, the samples may include vitals scores 110 as determined from the trained sequential RNN model 108, as described above. The viability score 114 may indicate the likelihood of infant survival over a predetermined period of time. This predetermined period may represent a threshold value for longer-term survival, as defined by medical literature and/or healthcare professionals. According to some embodiments of the present disclosure, the viability score 110 may be a numeric value on a scale of 1 to 10, where a lower score indicates a lower likelihood of survival, and a higher score indicates a greater likelihood. Alternatively, the viability score 114 may be an alpha character, ranging from A to Z, where A may represent a higher likelihood of survival, and Z representing a lower likelihood. In another alternative, the viability score 114 can be selected from a number of text descriptions indicating the likelihood of survival. For example, the viability score 114 may be one of, "high," "medium," and "low." Alternatively, some embodiments of the present disclosure may associate such textual descriptions with specific ranges of numeric and/or alpha values. In these ways, the viability score 114 can help clinicians and parents decide on what kind of active care to provide the pre-term infant.

According to some embodiments of the present disclosure, the system 100 may provide the viability score 114 offline and/or in real-time. Thus, in an offline implementation, the system 100 may provide the viability score 114 on-demand using continuous vital health parameters 102 within a predetermined time period. In this way, such embodiments may provide an indication of survival for a specific day, hour, week, and/or series thereof. Alternatively, in a real-time implementation, the system 100 may provide the viability score 114 using continuous vital health parameters 102 for a predetermined time period up to the current time. Additionally, such embodiments may calculate the viability score 114 at periodic intervals in real-time. In this way, it may be possible to analyze trends in the likelihood of survival in real-time.

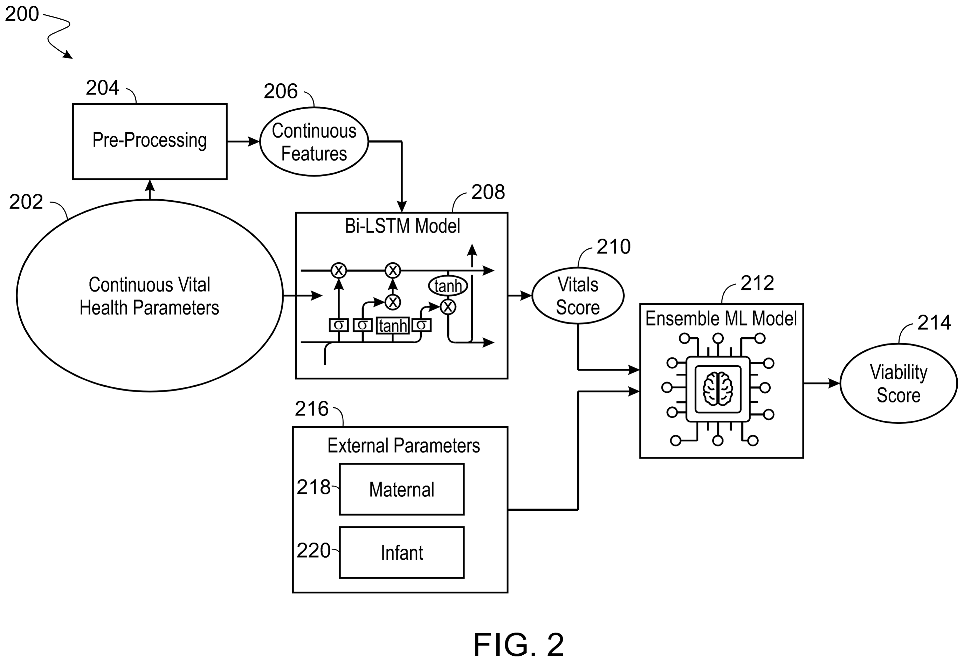

Fig. 2 is a data flow diagram of a system 200 for pre-term infant viability scoring according to one embodiment of the present disclosure. Similar to the system 100, the system 200 may input continuous vital health parameters 202 to pre-processing 204, which outputs (e.g., calculates) the continuous features 206.

The continuous vital health parameters 202 may be similar to the continuous vital health parameters 102 described with respect to Fig 1. Further, the pre-processing 204 may perform wavelet processing on the continuous vital health parameters 202. Additionally, the pre-processing 204 may be similar to the pre-processing 104, include linear and non-linear processing to generate the continuous features 206 that are input to a bi-directional long short-term memory (Bi-LSTM) model 208. Additionally, the continuous features 206 may include a feedback parameter. The Bi-LSTM model 208 may use the feedback parameter to determine how much feedback to use in its classification and/or prediction processes.

The Bi-LSTM model 208 is a recurrent neural network that processes data in forward and backward directions. More specifically, the Bi-LSTM model includes two LSTM models, one that processes the continuous vital health parameters 202 and continuous features 206 in a forward temporal direction, and one that processes this data in a backward temporal direction. According to some embodiments of the present disclosure, the Bi-LSTM model 208 may generate a vitals score 210 through classification or prediction. The vitals score 210 may be similar to the vitals score 110.

According to some embodiments of the present disclosure, the vitals score 210 may be input with external parameters 216 to an ensemble machine learning model 212, which may calculate a viability score 214. Typically, an ensemble machine learning model may combine the classifications of multiple models in an attempt to improve accuracy over the predication of a single machine learning model. However, in this example, the ensemble machine learning model 212 uses the output of the Bi-LSTM model 208 as an input, combined with an additional input (e.g., external parameters 216) in order to determine the viability of the pre-term infant. In this way, the ensemble model 212 performs a second classification to generate a viability score 214 that may provide a more accurate prediction of viability than the vitals score 210. The external parameters 216 may be similar to the external parameters 116 and may include maternal parameters 218 and infant parameters 220. The maternal parameters 218 and infant parameters 220 may be similar to the maternal parameters 118 and infant parameters 120.

According to some embodiments of the present disclosure, the ensemble machine learning model 212 may be trained to generate the viability score 214 based on the vitals score 210 provided by the sequential RNN model 208 and the external parameters 216. The viability score 214 may be similar to the viability score 114, and thus indicate the likelihood of long-term infant survival. According to some embodiments of the present disclosure, the ensemble machine learning model 212 may classify the viability score 114 with an ensemble type classifier, using an RUSBoost ensemble method, and a Bayesian optimization method. As stated previously, some embodiments of the present disclosure may provide the viability score 214 offline and/or in real-time. Accordingly, it may be possible to analyze trends in the likelihood of survival offline and/or in real-time.

Advantageously, using artificial intelligence (AI) to calculate the viability score 114, 214 with empirically determined inputs including vital traces (e.g., continuous vital health parameters 102, 202) of the pre-term infant; and linear, non-linear and/or wavelet processed signals (e.g., continuous features 106, 206), to the sequential recurrent neural network (RNN) may provide an objective estimation of the pre-term infant's likelihood of survival. Additionally, the incorporation of both the infant's health parameters and the mother's health parameters (e.g., external parameters 116, 216) as inputs into the machine learning model determining the viability score 114, 214 may provide additional objective factors that can help provide a more accurate estimation of pre-term infant viability. Further, estimating the viability score 114, 214 in real-time for pre-term infants with a gestational age of 22-32 weeks may provide further advantages by enabling parents and healthcare providers to react more quickly than possible with an offline system.

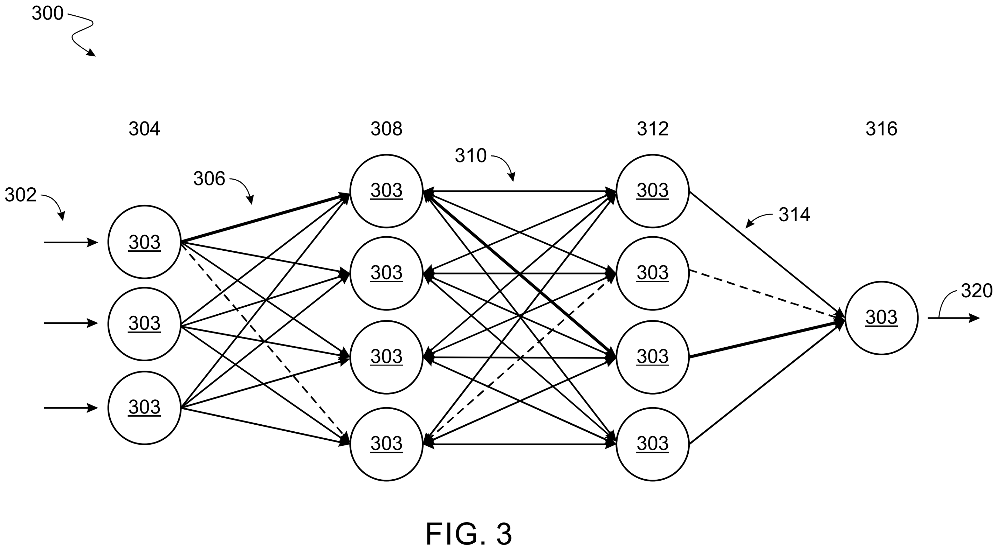

Fig. 3 is an example recurrent neural network (RNN) 300 according to some embodiments of the present disclosure. The RNN 300 may represent the RNN model 108 and Bi-LSTM model 208 described with respect to Figs. 1 and 2, respectively. Accordingly, the RNN 300 may receive inputs 302 (e.g., continuous vital health parameters 102 and continuous features 106), and generate output 320 (e.g., vitals score 110). In this example, the RNN 300 includes layers 304, 308, 312, and 316, each of which includes multiple nodes 303. The layer 304 is an input layer, layers 308 and 312 are hidden layers, and layer 316 is an output layer. While the example RNN 300 includes two hidden layers, some embodiments may include more or fewer hidden layers. Each input 302 corresponds to a node 303 of the input layer 304, and each node of the input layer 304 has a connection 306 to each node of hidden layer 308. Further, each node of hidden layer 308 has forward and backward connections 310 with each node of hidden layer 312. The backward connections 310 may be useful in retaining long term memory of both current features or previously identified features. Additionally, each node of the hidden layer 312 has a connection 314 to the output layer 316. Of connections 306, 310, and 314, the RNN 300 may assign varying weights to each of the connections 306, 310, 314 in the RNN 300.

Additionally, the input nodes (e.g., nodes 303 of the input layer 304) are activated through receipt of inputs 302. Further, nodes of hidden layers 308, 312 are activated through the flow of data through the RNN 300 via the connections 306 and 310, respectively. Additionally, the node 303 of the output layer 316 is activated after the RNN 300 sends data processed in hidden layers 308 and 312 via connections 314. When the node 303 of the output layer 316 is activated, the RNN 300 generates the output 320.

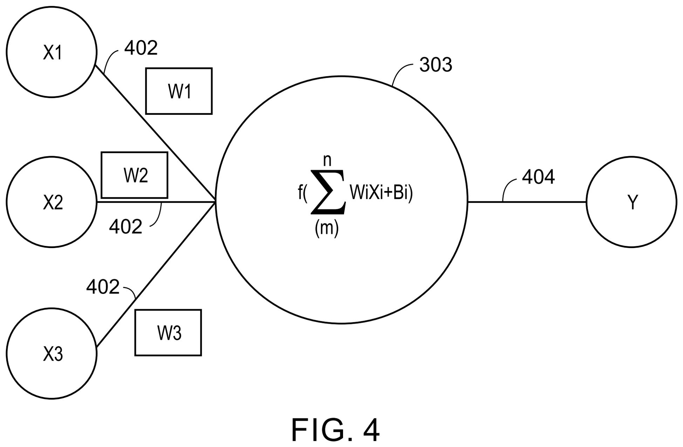

Fig. 4 is a diagram of an example neuron 303, described with respect to Fig. 3, in accordance with one embodiment of the present disclosure. In this example, the example neuron 303 includes multiple input connections 402. However, as stated previously, the example neuron may include more or fewer input connections 402. Each input connection 402 of example neuron 303 may be an output connection of a preceding neuron in the RNN 300. For example, in the case of forward data flow, the neurons of the hidden layer 308 may precede the neurons of the hidden layer 312. Additionally, in such a case, the neurons of the hidden layer 312 may be subsequent to the neurons of the hidden layer 308. While Fig. 4 depicts the neuron 303 as having a single output connection 404, the neuron 303 may have multiple output connections 404. Further, the neuron 303 may be data constructs, e.g., structures, instantiated class objects, matrices, and the like. In this example, the RNN 300 may provide weighted numerical values as input to the neuron 303 over connections 402 by applying weights W1, W2, W3, respectively to the inputs X1, X2, X3. The inputs X1, X2, X3 may include floating point or integer values. Further, the example neuron 303 may process the weighted inputs and send output Y over output connection 404. For example, the processing of an individual neuron 302 may be represented, generally, by EQUATION 1:

$$Y = f\left(\sum_1^n W_i X_i + B_i\right)$$
EQUATION 1

where n is the total number of input connections 402 to the neuron 303, and $B_i$ represents bias. The bias, $B_i$, is different from the bias discussed previously with respect to non-objective healthcare decisions. In contrast to that bias, the bias, $B_i$, may represent a predetermined constant that is added to the weighted sum, Y, which can provide an offset that enables the neuron 303 to activate even when the weighted sum, Y, is not otherwise large enough to activate the neuron 303. In some embodiments of the present disclosure, the value of Y may be based at least in part on whether the result of EQUATION 1 exceeds a predetermined threshold. In such a case, the neuron may assign a zero value to Y if the summation of the weighted inputs does not exceed a predetermined threshold.

Benefits of this system include reduced bias in care decisions, improved decision-making, enhanced fairness in healthcare, and filling a data gap. By providing an objective Viability Score, the solution minimizes the influence of personal biases, such as racial or social prejudices, in deciding the care for extreme pre-term infants. Healthcare professionals and parents can make more informed and data-driven decisions regarding active care, potentially improving outcomes for pre-term infants. By considering a broad range of factors, the solution promotes fairness and equity in neonatal care, enabling parents and healthcare providers to make care decisions for pre-term infants based on objective criteria rather than subjective judgments. The creation of a new dataset for 22-28 week pre-term infants contributes to the field by providing valuable data that can be used for further research and development, potentially leading to more effective interventions for this vulnerable group.

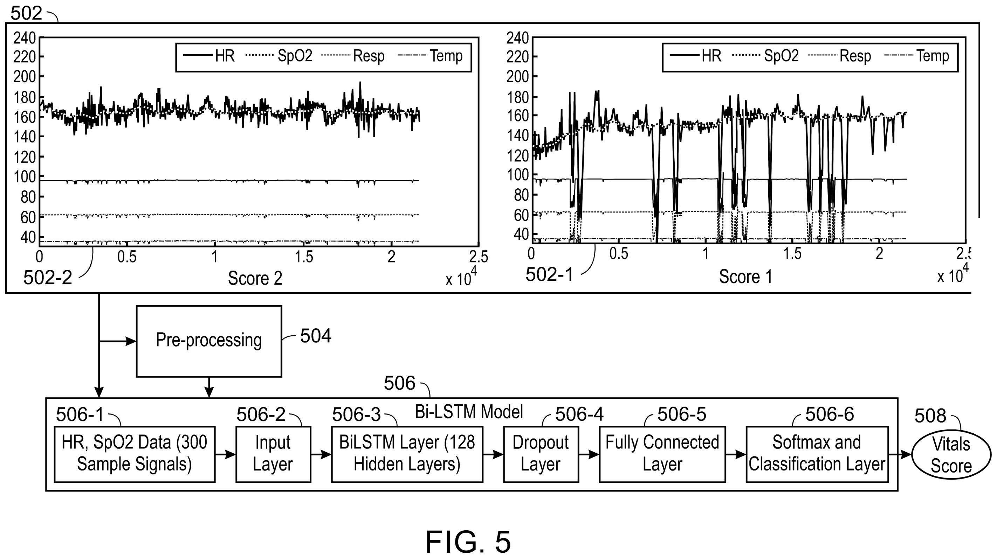

Fig. 5 is a diagram of a system 500 for vitals score training on a Bi-LSTM model 506 according to one embodiment of the present disclosure. The system includes training data 502, which may include multiple labeled samples of vital health parameters in sets of five, where each set includes labeled continuous vital health parameters 102, 202 for scores of one through five. However, for the purpose of clarity, samples for only two scores are shown in this example. Further, the continuous vital health parameters shown represent four samples per second. Thus, for a duration of 90 minutes, total samples may be in the range of (90 minutes * 4 samples per second * 60 seconds per minute) = 21,600. Accordingly, these examples are represented as in the order of $10^4$ samples. More specifically, the labeled vital health parameters 502-1, 502-2 are represented in graphs, and labeled with a vitals score. The labeled vital health parameters 502-1, 502-2 include continuous heart rate (HR), oxygen saturation (SpO2), respiration (Resp), and temperature (Temp), graphed over a time period of 2.5 hours. Further, the labeled vital health parameters 502-1, 502-2 include vitals score labels of 1 and 2, respectively. Accordingly, the system 500 may input the training data 502 to a Bi-LSTM model 508, which may be similar to the Bi-LSTM model 208, described with respect to Fig. 2. Additionally, the system 500 may input the training data 502 to pre-processing 504, which may be similar to the pre-processing 104, 204, described with respect to Figs. 1, 2. As stated previously, the pre-processing 504 may perform linear /non-linear processing or wavelet processing or actual vital parameter signals to generate continuous features 506. The continuous features 506 may be similar to the continuous features 106, 206, and may be input to Bi-LSTM model 508.

In this example, the Bi-LSTM model 508 includes the training data 502 and continuous features 506 in the heart rate (HR), oxygen saturation (SpO2), respiration (Resp), and temperature (Temp) data 506-1, which may be input to input layer 508-2. Alternatively, or additionally, the continuous features (e.g., continuous features 206) may be input to the input layer 508-2. The input layer 508-2 may be similar to the input layer 304, described with respect to Fig. 3. Further, the input layer 506-2 may process the HR, SpO2, respiration, and temperature data 508-1, and/or their continuous features (e.g., continuous features 106, 206). Additionally, the input layer 506-2 may forward the processed data to the Bi-LSTM layer 508-3. The Bi-LSTM layer 508-3 may include hidden layers, similar to the hidden layers 308, 312, described with respect to Fig. 3. According to some embodiments of the present disclosure, the Bi-LSTM layer 508-3 may include 2 or more hidden layers. Further, the Bi-LSTM model 506 may forward the processed data from the Bi-LSTM layer 506-3 to a dropout layer 506-4. In order to mitigate overfitting, and reduce the Bi-LSTM model's 506 reliance on specific neurons (e.g., neurons 303), the dropout layer 506-4 may randomly drop out a predetermined percentage of neurons during training. According to some embodiments of the present disclosure, this predetermined percentage may be based on a feedback parameter provided by an operator of the system 500. Further, the dropout layer 506-4 may forward the data from the remaining neurons to a fully-connected layer 506-5. The fully-connected layer 506-5 may further process the combined output from the dropout layer 508-3 in forward and backward directions. Additionally, the Bi-LSTM model 506 may forward the data from the fully-connected layer 506-5 to the softmax and classification layer 506-6. The softmax and classification layer 506-6 may calculate a set of vitals scores based on the outputs from the fully-connected layer. Additionally, the softmax and classification layer 506-6 may perform softmax activation. Performing softmax activation may involve calculating a probability distribution for the calculated vitals scores. Additionally, the Bi-LSTM model 506 may provide the vitals scores 508 for each of the labeled vital health parameters. The vitals scores 508 may be similar to the vitals score 110, 210, described with respect to Figs. 1, 2. According to some embodiments of the present disclosure, the Bi-LSTM model 506 may select the vitals score 508 based on the vitals score with the greatest probability, as determined by the softmax activation.

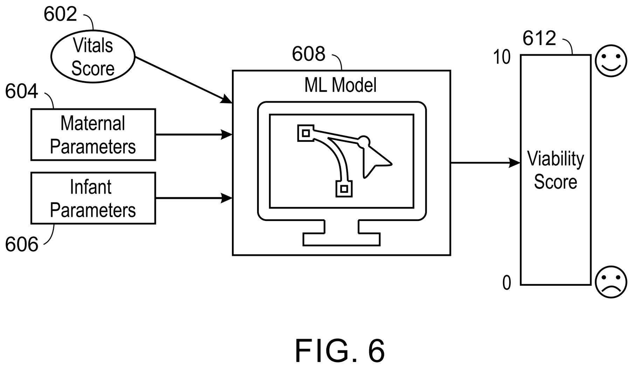

Fig. 6 is a data flow diagram of a system 600 for calculating a viability score 612 for pre-term infants according to one embodiment of the present disclosure. According to some embodiments of the present disclosure, the machine learning model (ML) 608 may use a vitals score 602, maternal parameters 604, and infant parameters 606, as inputs, to calculate the viability score 612. In one example, the Bi-LSTM model 208 may generate the vitals score 602 in real-time based on continuous vitals health parameters 202 and continuous features 206 as calculated by the pre-processing 204, described with respect to Fig. 2. Further, the maternal parameters 604 and infant parameters 606 may be similar to the external parameters 116, 216 (i.e., maternal parameters 118, 218 and infant parameters 120, 220), described with respect to Figs. 1, 2.

In this example, the viability score 612 may be a numeric value ranging on a scale from 1 to 10. In one embodiment of the present disclosure, a viability score 612 on the lower side of the scale may indicate a higher risk of infant mortality and morbidity. Conversely, a viability score 612 on the higher side of the scale may represent a higher chance of survival, leading to happier outcomes. According to some embodiments of the present disclosure, the ML model 608 may be an ensemble model. As stated previously, an ensemble machine learning model (e.g., ensemble machine learning model 212) uses the output of the Bi-LSTM model 208 (e.g., vitals score 210) as an input, combined with an additional input (e.g., external parameters 216) in order to determine the viability of the pre-term infant. In this way, an ensemble model performs a second classification to generate a viability score 612 that may provide a more accurate prediction of viability than the vitals score 602.

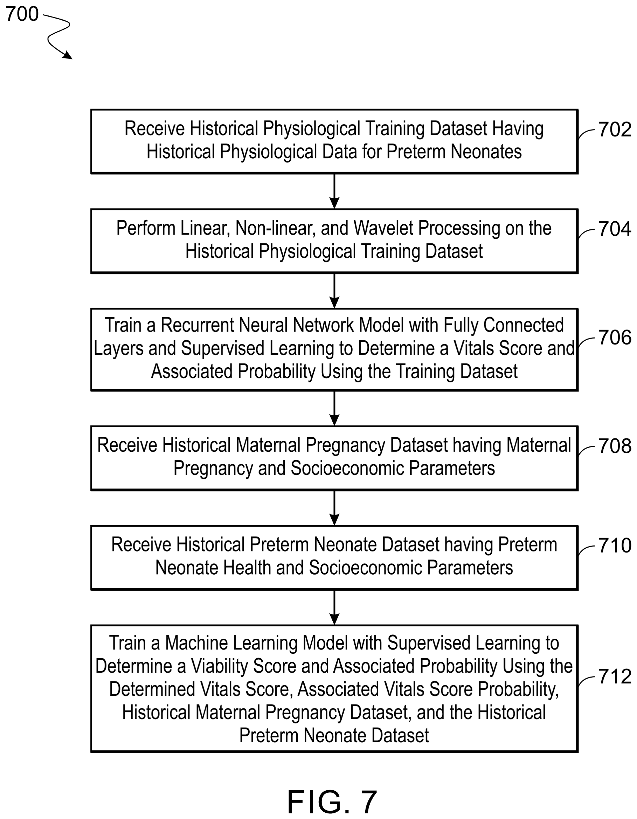

Fig. 7 is a flow chart of a method 700 for pre-term infant viability scoring according to one embodiment of the present disclosure. The method 700 may be performed by a viability scoring manager.

At operation 702, the viability scoring manager may receive training data for pre-term infants. The training data (e.g., training data 502) may include historical physiological data (i.e., continuous vitals health parameters) for pre-term infants with positive and negative longer and shorter-term outcomes. A positive outcome may represent the long-term survival (e.g., survival into adulthood) of the pre-term infant. Conversely, a negative outcome may represent morbidity and/or mortality of the pre-term infant while still a patient in the NICU.

At operation 704, the viability scoring manager may pre-process the training data 502. As stated previously, pre-processing the training data may involve performing linear/non-linear, and wavelet processing on the training data 502, similar to the pre-processing 104, 204, described with respect to Figs. 1, 2. In this way, the viability scoring manager may generate continuous features of the pre-term infant's continuous vital health parameters (e.g., continuous features 106, 206).

At operation 706, the viability scoring manager may train a sequential recurrent neural network model (e.g., sequential RNN model 108, Bi-LSTM model 208, 508) to calculate a vitals score (e.g., vitals score 110, 210). According to some embodiments of the present disclosure, the sequential RNN model may include fully connected layers, and may be trained using supervised learning, as described with respect to Figs. 1, 2, 5.

At operation 708, the viability scoring manager may receive a training dataset for a machine learning model that calculates a viability score. The training dataset may include historical vitals scores (e.g., vitals scores 510), and external parameters. As stated previously, the external parameters may include maternal and infant parameters (e.g., maternal parameters 118, 218 and infant parameters 120, 220).

At operation 710, the viability scoring manager may train a machine learning model (e.g., ensemble model 212) with supervised learning to calculate a viability score (e.g., viability score 120, 220). Training the machine learning model may be similar to the training described with respect to Fig. 1. As stated previously, the viability score 120, 220 may be associated with a probability that the viability score 120, 220 accurately represents an outcome for the pre-term infant.

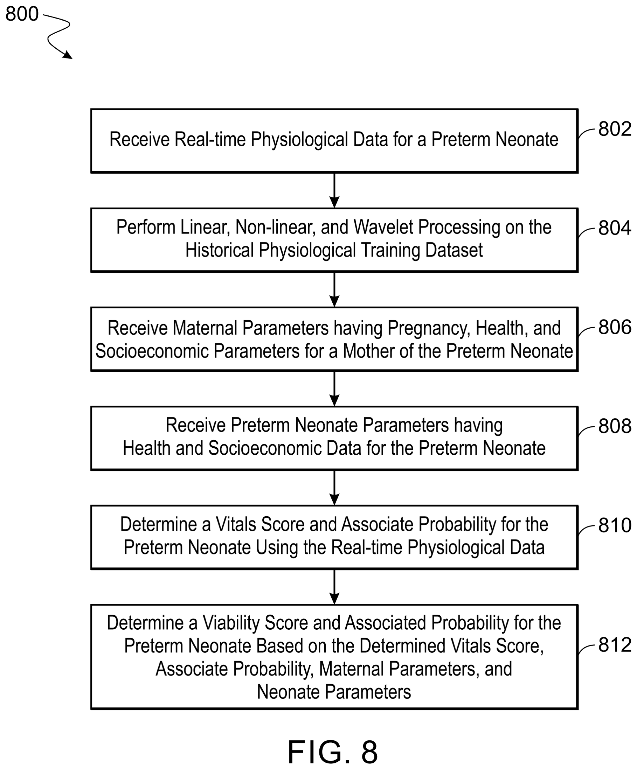

Fig. 8 is a flow chart of a method 800 for pre-term infant viability scoring according to one embodiment of the present disclosure. The method 800 may be performed by a viability scoring manager.

At operation 802, the viability scoring manager may receive real-time physiological data for a pre-term infant. The real-time physiological data may include the continuous vital health parameters 102, 202, for a pre-determined time period.

At operation 804, the viability scoring manager may perform pre-processing on the real-time physiological data. Performing pre-processing may be similar to the pre-processing 104, 204, described with respect to Figs. 1, 2, and 7.

At operation 806, the viability scoring manager may receive external parameters for the mother and infant. As stated previously, the external parameters (e.g., external parameters 116, 216) may include maternal parameters 118, 218 and infant parameters 120, 220.

At operation 808, the viability scoring manager may determine a vitals score using a sequential RNN model (e.g., sequential RNN model 108, Bi-LSTM model 208) with inputs of real-time physiological data and continuous features. The real-time physiological data may include the continuous vital health parameters 102, 202, and continuous features may include continuous features 106, 206. As stated previously, the vitals score (e.g., vitals score 110, 210, 510) may represent how healthy the pre-term infant is based on the continuous vital health parameters 102, 202. For example, a pre-term infant with steady (e.g., non-volatile) continuous vital health parameters 102, 202 may be more healthy than a pre-term infant with inconsistent, or erratic vital continuous vital health parameters 102, 202. Accordingly, the more healthy pre-term infant may have a higher vitals score 110, 210 than the less healthy pre-term infant.

At operation 810, the viability scoring manager may calculate a viability score 114, 214 for the preterm infant based using a machine learning model with inputs of the vitals score 110, 210, 510, and external parameters 116, 216. As stated previously, the viability score 114, 214 may be a numeric, alphanumeric, and/or textual value indicating the likelihood of survival for the pre-term infant. According to one embodiment of the present disclosure, the viability score 114, 214 may be a numeric value ranging from 1 to 10, where a 1 indicates a lower likelihood of survival (e.g., a higher likelihood of morbidity and/or mortality) and a 10 indicates a higher likelihood of survival.

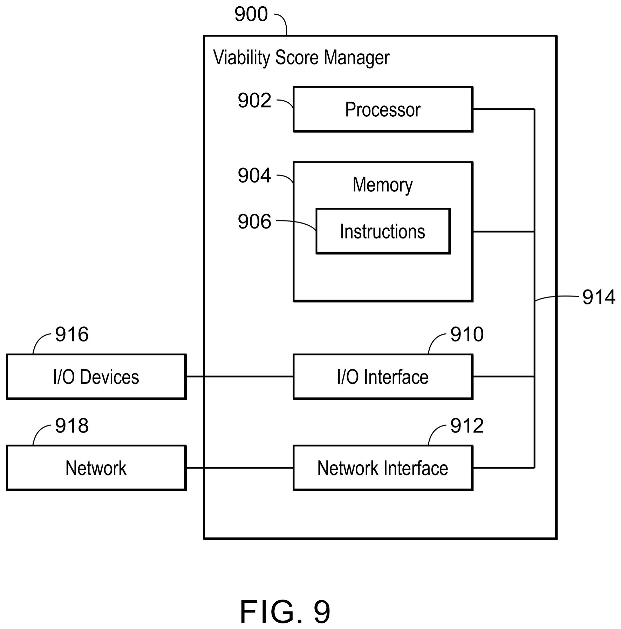

Fig. 9 is an exemplary viability scoring manager according to one embodiment of the present disclosure. In this example, the viability scoring manager 900 includes a processor 902, memory 904, input-output (I/O) interface 908, and network interface 910, which may be connected by an interconnect 912. The processor 902 may be a computer processing circuit (e.g., a central processing unit (CPU)) that retrieves and executes programming instructions 906 stored in the memory 904 to perform the functionality described herein. More specifically, the instructions 906 may cause the processor 902 to perform the functionality described with respect to Figs. 1 through 8. The interconnect 912 may move data, such as programming instructions, between the processor 902, memory 904, I/O interface 908, and network interface 910. The interconnect 912 may include one or more buses.

The memory 904 may be a computer memory or storage device, including volatile memory, such as a random access memory (RAM) device (e.g., static RAM, dynamic RAM, and the like), non-volatile memory, such as a hard disk drive, solid state device (SSD), removable memory cards, optical storage, flash memory devices, and the like. In some examples, the memory 904 may include volatile and non-volatile memory devices. Further, the memory 904 may store instructions 906.

Additionally, the viability scoring manager 900 may be in electronic communication with I/O devices 914 through the I/O interface 908, and with a network 916 through the network interface 910. The I/O devices 914 may capture inputs and provide outputs as described herein. The network 916 may be an electronic communication network, such as a local area network, wide area network, and the like, for processing communications between the viability scoring manager 900 and the machine learning models described herein. In some examples, the network 916 may be wired, wireless (e.g., wi-fi, Bluetooth, or cellular), or some other computer communication network.

In some embodiments, the viability scoring manager 900 may be a server computer or similar device without a user interface but which receives requests from other computer systems having one or more user interfaces. Further, in some embodiments, the viability scoring manager 900 may be a portable computer, laptop, tablet computer, pocket computer, telephone, smart phone, or the like.

An example system includes a processor and a memory. The memory includes instructions executable by the processor to receive training data having vitals scores for pre-term infants indicating a healthiness of a pre-term infant having specific vitals measurements. Further, the instructions are executable to receive training data having maternal health parameters and pregnancy parameters for pregnant women that gave birth to pre-term infants. Additionally, the instructions are executable to receive training data having pre-term infant health parameters and birth parameters for the pre-term infants. Further, the instructions are executable to train an artificial intelligence model to infer a viability score by using the maternal parameters training data, pre-term parameters training data, pre-term vitals training data, and supervised learning. Additionally, the viability score indicates a likelihood of survival for a pre-term infant having a vitals score, maternal parameters, and pre-term parameters.

In an example, the viability score further indicates a likelihood of morbidity and mortality.

In an example, the instructions executable by the processor to receive a pre-term vitals training dataset having vitals measurements for pre-term infants. Additionally, the instructions are executable by the processor to perform linear processing on the pre-term vitals training dataset. Further, , the instructions are executable by the processor to perform non-linear processing on the pre-term vitals training dataset. Additionally, the instructions are executable by the processor to perform wavelet processing on the pre-term vitals training dataset. Further, the instructions are executable by the processor to train a recurrent neural network model to infer a vitals score by using the pre-term vitals training dataset and supervised learning. Additionally, the vitals score indicates a healthiness of a pre-term infant having specific vitals measurements.

In an example, the recurrent neural network model includes a bidirectional long short-term memory model.

In an example, the instructions are executable by the processor to receive a heart rate measurements that are measured over a specific period of time. Additionally, the instructions are executable by the processor to receive oxygen saturation measurements that are measured over the specific period of time or continuously. Further, the instructions are executable by the processor to receive respiration rate measurements that are measured over the specific period of time or continuously. Additionally, the instructions are executable by the processor to receive body temperature measurements that are measured over the specific period of time or continuously. Further, the instructions are executable by the processor to perform linear processing on the heart rate measurements, oxygen saturation measurements, respiration rate measurements, and body temperature measurements. Additionally, the instructions are executable by the processor to perform non-linear processing on the heart rate measurements, oxygen saturation measurements, respiration rate measurements, and body temperature measurements. Further, the instructions are executable by the processor to perform wavelet processing on the heart rate measurements, oxygen saturation measurements, respiration rate measurements, and body temperature measurements. Additionally, the instructions are executable by the processor to infer a real-time vitals score for the pre-term infant using the trained deep learning model having inputs including: the heart rate measurements, the oxygen saturation measurements, the respiration rate measurements, and the body temperature measurements.

In an example, the instructions are executable by the processor to receive the real-time vitals score for the pre-term infant. Additionally, the instructions are executable by the processor to receive maternal parameters including individual maternal health parameters, and individual pregnancy parameters for a mother that gave birth to the pre-term infant. Further, the instructions are executable by the processor to receive pre-term infant parameters including individual pre-term health parameters, and individual birth parameters for the pre-term infant. Additionally, the instructions are executable by the processor to infer a viability score for the pre-term infant using the machine learning model having inputs including: the real-time vitals score, the maternal parameters, and the pre-term infant parameters.

In an example, the instructions are executable by the processor to present the viability score in real-time using an electronic device.

An example method includes receiving a vitals score for a pre-term infant, the vitals score indicating a healthiness of the pre-term infant having specific vitals measurements. Additionally, the method includes receiving maternal health parameters and pregnancy parameters for a mother that gave birth to the pre-term infant. Further, the method includes receiving pre-term infant health parameters and birth parameters for the pre-term infant. Additionally, the method includes inferring a viability score for the pre-term infant by using a machine learning model trained using a maternal parameters training dataset, a pre-term parameters training dataset, a pre-term vitals training dataset, and supervised learning. Further, the machine learning model includes inputs comprising the vitals score, the maternal health parameters, and the pre-term infant health parameters. Additionally, the viability score indicates a likelihood of survival for a pre-term infant having the vitals score, the maternal health parameters, and the pre-term infant health parameters. Further, the pre-term vitals training dataset includes training vitals scores for pre-term infants. Additionally, the maternal parameters training dataset includes maternal health parameters and pregnancy parameters for pregnant women that gave birth to the pre-term infants. Further, the pre-term parameters training dataset includes pre-term infant health parameters and birth parameters for the pre-term infants.

In an example, the method includes receiving heart rate measurements that are measured over a specific period of time. Further, the method includes receiving oxygen saturation measurements that are measured over the specific period of time. Additionally, the method includes receiving respiration rate measurements that are measured over the specific period of time. Further, the method includes receiving body temperature measurements that are measured over the specific period of time. Additionally, the method includes performing linear processing on the heart rate measurements, oxygen saturation measurements, respiration rate measurements, and body temperature measurements. Further, the method includes performing non-linear processing on the heart rate measurements, oxygen saturation measurements, respiration rate measurements, and body temperature measurements. Additionally, the method includes performing wavelet processing on the heart rate measurements, oxygen saturation measurements, respiration rate measurements, and body temperature measurements. Further, the method includes inferring the vitals score for the pre-term infant by using a recurrent neural network model trained with a pre-term vitals training dataset comprising vitals measurements for pre-term infants. Additionally, the recurrent neural network model has inputs including the heart rate measurements, the oxygen saturation measurements, respiration rate measurements, and the body temperature measurements.

An example computer-readable storage medium includes instructions that are executable by a processor to receive a vitals score for a pre-term infant. Additionally, the vitals score indicates a healthiness of a pre-term infant having specific vitals measurements. Further, the instructions are executable by the processor to receive maternal health parameters and pregnancy parameters for a mother that gave birth to the pre-term infant. Additionally, the instructions are executable by the processor to receive pre-term infant health parameters and birth parameters for the pre-term infant. Further, the instructions are executable by the processor to infer a viability score for the pre-term infant by using a machine learning model trained using a maternal parameters training dataset, a pre-term parameters training dataset, a pre-term vitals training dataset, and supervised learning. Additionally, the machine learning model includes inputs having the vitals score, the maternal health parameters, and the pre-term infant health parameters. Further, the viability score indicates a likelihood of survival for a pre-term infant having the vitals score, the maternal health parameters, and the pre-term infant health parameters. Additionally, the pre-term vitals training dataset includes training vitals scores for pre-term infants. Further, the maternal parameters training dataset includes maternal health parameters and pregnancy parameters for pregnant women that gave birth to the pre-term infants. Additionally, the pre-term parameters training dataset includes pre-term infant health parameters and birth parameters for the pre-term infants.

It should be noted that, as used herein, the term mechanism can encompass hardware, software, firmware, or any suitable combination thereof. In some embodiments, any suitable computer readable media can be used for storing instructions for performing functions and/or processes described herein. For example, in some embodiments, computer readable media can be transitory or non-transitory. For example, non-transitory computer readable media can include media such as magnetic media (such as hard disks, floppy disks, etc.), optical media (such as compact discs, digital video discs, Blu-ray discs, etc.), semiconductor media (such as RAM, Flash memory, electrically programmable read only memory (EPROM), electrically erasable programmable read only memory (EEPROM), etc.), any suitable media that is not fleeting or devoid of any semblance of permanence during transmission, and/or any suitable tangible media. As another example, transitory computer readable media can include signals on networks, in wires, conductors, optical fibers, circuits, or any suitable media that is fleeting and devoid of any semblance of permanence during transmission, and/or any suitable intangible media.

As used herein, the term, mechanism, can encompass hardware, software, firmware, or any suitable combination thereof. In some embodiments, any suitable computer readable media can be used for storing instructions for performing functions and/or processes described herein. For example, in some embodiments, computer readable media can be transitory or non-transitory. For example, non-transitory computer readable media can include media such as magnetic media (such as hard disks, floppy disks, etc.), optical media (such as compact discs, digital video discs, Blu-ray discs, etc.), semiconductor media (such as RAM, Flash memory, electrically programmable read only memory (EPROM), electrically erasable programmable read only memory (EEPROM), etc.), any suitable media that is not fleeting or devoid of any semblance of permanence during transmission, and/or any suitable tangible media. As another example, transitory computer readable media can include signals on networks, in wires, conductors, optical fibers, circuits, or any suitable media that is fleeting and devoid of any semblance of permanence during transmission, and/or any suitable intangible media.

This written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

**Claims**

1. A system, comprising:

    a processor; and
    a memory comprising instructions executable by the processor to:

        receive a pre-term vitals training dataset comprising a plurality of training vitals scores for a plurality of pre-term infants, the training vitals scores indicating a healthiness of a pre-term infant having specific vitals measurements;
        receive a maternal parameters training dataset comprising maternal health parameters and pregnancy parameters for a plurality of pregnant women that gave birth to the plurality of pre-term infants;
        receive a fetal parameters training dataset comprising pre-term infant health parameters and birth parameters for the plurality of pre-term infants; and
        train an artificial intelligence model, comprising a deep learning model and a machine learning model, to infer a viability score by using the maternal parameters training dataset, the pre-term parameters training dataset, the pre-term vitals training dataset, and supervised learning, the viability score indicating a likelihood of survival for a pre-term infant having a specific vitals score, specific maternal parameters, and specific pre-term parameters.

2. The system of claim 1, the viability score further indicating a likelihood of morbidity and mortality.

3. The system of claim 1, the instructions executable by the processor to:

    receive a pre-term vitals training dataset comprising a plurality of vitals measurements for a plurality of pre-term infants;
    perform linear processing on the pre-term vitals training dataset;
    perform non-linear processing on the pre-term vitals training dataset;
    perform wavelet processing on the pre-term vitals training dataset; and
    train a recurrent neural network model to infer a vitals score by using the pre-term vitals training dataset and supervised learning, the vitals score indicating a healthiness of a pre-term infant having specific vitals measurements.

4. The system of claim 3, the recurrent neural network model having fully connected layers.

5. The system of claim 3, the machine learning model comprising an ensemble machine learning model.

6. The system of claim 3, the recurrent neural network model comprising a bidirectional long short-term memory model.

7. The system of claim 1, the instructions executable by the processor to:

    receive a plurality of heart rate measurements that are measured continuously;
    receive a plurality of oxygen saturation measurements that are measured continuously;
    receive a plurality of respiration rate measurements that are measured continuously;
    receive a plurality of body temperature measurements that are measured continuously;
    perform linear processing on the heart rate measurements, oxygen saturation measurements, respiration rate measurements, and body temperature measurements;
    perform non-linear processing on the heart rate measurements, oxygen saturation measurements, respiration rate measurements, and body temperature measurements;
    perform wavelet processing on the heart rate measurements, oxygen saturation measurements, respiration rate measurements, and body temperature measurements; and
    infer a real-time vitals score for the pre-term infant using the trained deep learning model having inputs comprising: the heart rate measurements, the oxygen saturation measurements, the respiration rate measurements, and the body temperature measurements.

8. The system of claim 7, the instructions executable by the processor to:

receive the real-time vitals score for the pre-term infant;
receive a plurality of maternal parameters comprising individual maternal health parameters, and individual pregnancy parameters for a mother that gave birth to the pre-term infant;
receive a plurality of pre-term infant parameters comprising individual pre-term health parameters, and individual birth parameters for the pre-term infant; and
infer a viability score for the pre-term infant using the machine learning model having inputs comprising: the real-time vitals score, the maternal parameters, and the pre-term infant parameters.

9. The system of claim 8, the instructions executable by the processor to present the viability score in real-time using an electronic device.

10. A method, comprising:

receiving a vitals score for a pre-term infant, the vitals score indicating a healthiness of the pre-term infant having specific vitals measurements;
receiving a plurality of maternal health parameters and a plurality of pregnancy parameters for a mother that gave birth to the pre-term infant;
receiving a plurality of pre-term infant health parameters and a plurality of birth parameters for the pre-term infant; and
inferring a viability score for the pre-term infant by using a machine learning model trained using a maternal parameters training dataset, a pre-term parameters training dataset, a pre-term vitals training dataset, and supervised learning, the machine learning model having inputs comprising the vitals score, the maternal health parameters, and the pre-term infant health parameters, the viability score indicating a likelihood of survival for a pre-term infant having the vitals score, the maternal health parameters, and the pre-term infant health parameters, the pre-term vitals training dataset comprising a plurality of training vitals scores for a plurality of pre-term infants, the maternal parameters training dataset comprising maternal health parameters and pregnancy parameters for a plurality of pregnant women that gave birth to the plurality of pre-term infants, and the pre-term parameters training dataset comprising pre-term infant health parameters and birth parameters for the plurality of pre-term infants.

11. The method of claim 10, the viability score further indicating a likelihood of morbidity and mortality.

12. The method of claim 10, comprising:

receiving a plurality of heart rate measurements that are measured over a specific period of time;
receiving a plurality of oxygen saturation measurements that are measured over the specific period of time;
receiving a plurality of respiration rate measurements that are measured over the specific period of time;
receiving a plurality of body temperature measurements that are measured over the specific period of time;
performing linear processing on the heart rate measurements, oxygen saturation measurements, respiration rate measurements, and body temperature measurements;
performing non-linear processing on the heart rate measurements, oxygen saturation measurements, respiration rate measurements, and body temperature measurements;
performing wavelet processing on the heart rate measurements, oxygen saturation measurements, respiration rate measurements, and body temperature measurements; and
inferring the vitals score for the pre-term infant by using a recurrent neural network model trained with a pre-term vitals training dataset comprising a plurality of vitals measurements for a plurality of pre-term infants, and the recurrent neural network model having inputs comprising the heart rate measurements, the oxygen saturation measurements, respiration rate measurements, and the body temperature measurements.

13. The method of claim 12, the recurrent neural network model having fully connected layers.

14. The method of claim 12, the machine learning model comprising an ensemble machine learning model and the recurrent neural network model comprising a bidirectional long short-term memory model.

FIG. 1

FIG. 2

FIG. 3

$$f(\sum_{(m)}^{n} WiXi+Bi)$$

FIG. 4

FIG. 5

EP 4 787 385 A1

602 Vitals Score

604 Maternal Parameters

606 Infant Parameters

608 ML Model

612 Viability Score

10

0

FIG. 6

700

| Receive Historical Physiological Training Dataset Having Historical Physiological Data for Preterm Neonates | 702 |

↓

| Perform Linear, Non-linear, and Wavelet Processing on the Historical Physiological Training Dataset | 704 |

↓

| Train a Recurrent Neural Network Model with Fully Connected Layers and Supervised Learning to Determine a Vitals Score and Associated Probability Using the Training Dataset | 706 |

↓

| Receive Historical Maternal Pregnancy Dataset having Maternal Pregnancy and Socioeconomic Parameters | 708 |

↓

| Receive Historical Preterm Neonate Dataset having Preterm Neonate Health and Socioeconomic Parameters | 710 |

↓

| Train a Machine Learning Model with Supervised Learning to Determine a Viability Score and Associated Probability Using the Determined Vitals Score, Associated Vitals Score Probability, Historical Maternal Pregnancy Dataset, and the Historical Preterm Neonate Dataset | 712 |

## FIG. 7

800

Receive Real-time Physiological Data for a Preterm Neonate — 802

↓

Perform Linear, Non-linear, and Wavelet Processing on the Historical Physiological Training Dataset — 804

↓

Receive Maternal Parameters having Pregnancy, Health, and Socioeconomic Parameters for a Mother of the Preterm Neonate — 806

↓

Receive Preterm Neonate Parameters having Health and Socioeconomic Data for the Preterm Neonate — 808

↓

Determine a Vitals Score and Associate Probability for the Preterm Neonate Using the Real-time Physiological Data — 810

↓

Determine a Viability Score and Associated Probability for the Preterm Neonate Based on the Determined Vitals Score, Associate Probability, Maternal Parameters, and Neonate Parameters — 812

# FIG. 8

900

Viability Score Manager

902 — Processor

904 — Memory

906 — Instructions

914

916 — I/O Devices

910 — I/O Interface

918 — Network

912 — Network Interface

FIG. 9

Europäisches
Patentamt

European
Patent Office

Office européen
des brevets

# EUROPEAN SEARCH REPORT

Application Number

EP 26 15 0502

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2023/164308 A2 (UNIV LELAND STANFORD JUNIOR [US]) 31 August 2023 (2023-08-31) | 1-14 | INV.<br>G16H50/30 |
| Y | * The whole document, in particular: Paragaphs [0046], [0073] - [0075], [0080], [0083] - [0085], [0116], Table 1, Figure 9. * | 3-9, 12-14 | G16H50/20<br>A61B5/00<br>G06N3/0442<br>G16H50/70 |
| | ----- | | |
| X | PODDA MARCO ET AL: "A machine learning approach to estimating preterm infants survival: development of the Preterm Infants Survival Assessment (PISA) predictor",<br>SCIENTIFIC REPORTS,<br>vol. 8, no. 1,<br>13 September 2018 (2018-09-13),<br>XP093400209,<br>US<br>ISSN: 2045-2322, DOI:<br>10.1038/s41598-018-31920-6 | 1-14 | |
| Y | * The whole document, in particular:<br>Pages 2 - 3, 8, Table 1. * | 3-9, 12-14 | TECHNICAL FIELDS SEARCHED (IPC) |
| | ----- | | |
| X | TEJI JAGJIT S. ET AL: "NeoAI 1.0: Machine learning-based paradigm for prediction of neonatal and infant risk of death",<br>COMPUTERS IN BIOLOGY AND MEDICINE NEW YORK, NY, US,<br>vol. 147, 18 May 2022 (2022-05-18),<br>XP087113743,<br>ISSN: 0010-4825, DOI:<br>10.1016/J.COMPBIOMED.2022.105639 | 1-14 | G16H<br>G06E<br>A61B<br>G06N |
| Y | * The whole document, in particular:<br>Pages 1 - 3, Table 1. * | 3-9, 12-14 | |
| | ----- | | |
| | -/-- | | |

The present search report has been drawn up for all claims

| Place of search | Date of completion of the search | Examiner |
|---|---|---|
| Munich | 17 June 2026 | Henschel, Leonie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

    ........................................................................
& : member of the same patent family, corresponding document

EPO FORM 1503 03.82 (P04C01)

| | Europäisches Patentamt | EUROPEAN SEARCH REPORT | Application Number |
|---|---|---|---|
| | European Patent Office | | EP 26 15 0502 |
| | Office européen des brevets | | |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | BAKER STEPHANIE ET AL: "Continuous and automatic mortality risk prediction using vital signs in the intensive care unit: a hybrid neural network approach", SCIENTIFIC REPORTS, vol. 10, no. 1, 4 December 2020 (2020-12-04), XP093400468, US ISSN: 2045-2322, DOI: 10.1038/s41598-020-78184-7 * The whole document, in particular: Pages 1, 3, 5. * | 3-9, 12-14 | |
| | ----- | | |
| | | | TECHNICAL FIELDS SEARCHED (IPC) |

The present search report has been drawn up for all claims

| Place of search | Date of completion of the search | Examiner |
|---|---|---|
| Munich | 17 June 2026 | Henschel, Leonie |

EPO FORM 1503 03.82 (P04C01)

## ANNEX TO THE EUROPEAN SEARCH REPORT
## ON EUROPEAN PATENT APPLICATION NO.

EP 26 15 0502

This annex lists the patent family members relating to the patent documents cited in the above-mentioned European search report.
The members are as contained in the European Patent Office EDP file on
The European Patent Office is in no way liable for these particulars which are merely given for the purpose of information.

17-06-2026

| Patent document cited in search report | Publication date | Patent family member(s) | | Publication date |
|---|---|---|---|---|
| WO 2023164308 A2 | 31-08-2023 | AU | 2023225811 A1 | 12-09-2024 |
| | | CA | 3253411 A1 | 31-08-2023 |
| | | CN | 119137680 A | 13-12-2024 |
| | | EP | 4487348 A2 | 08-01-2025 |
| | | US | 20260120865 A1 | 30-04-2026 |
| | | WO | 2023164308 A2 | 31-08-2023 |

EPO FORM P0459

For more details about this annex : see Official Journal of the European Patent Office, No. 12/82